# EUROPEAN PATENT SPECIFICATION

(11) **EP 4 433 002 B1**
(45) Date of publication and mention of the grant of the patent: **18.11.2026**
(21) Application number: 22801551.7
(22) Date of filing: 26.10.2022
(51) Int. Cl.: A61F 9/008

(54) **IMAGING A TARGET WITHIN AN EYE AND CALIBRATING IMAGING DEVICES**
ABBILDUNG EINES ZIELS IN EINEM AUGE UND KALIBRIERUNG VON ABBILDUNGSVORRICHTUNGEN
IMAGERIE D'UNE CIBLE À L'INTÉRIEUR D'UN OEIL ET ÉTALONNAGE DE DISPOSITIFS D'IMAGERIE

(30) Priority: 19.11.2021 US 202163281487 P
(43) Date of publication of application: 25.09.2024
(73) Proprietor: Alcon Inc., 1701 Fribourg (CH)
(72) Inventor: SHARMA, Utkarsh, Solon, Ohio 44139 (US); BOR, Zsolt, Lake Forest, California 92630 (US)
(74) Representative: Murgitroyd & Company
(86) International application number: PCT/IB2022/060313
(87) International publication number: WO 2023/089419

(56) References cited:
- US-A1- 2020 038 241
- US-A1- 2020 129 336
- MILSTON REBECCA ET AL: "Vitreous floaters: Etiology, diagnostics, and management", SURVEY OF OPHTHALMOLOGY, vol. 61, no. 2, 8 December 2015 (2015-12-08), pages 211 - 227, XP029439479, ISSN: 0039-6257, DOI: 10.1016/J.SURVOPHTHAL.2015.11.008
- NICUSOR IFTIMIA ET AL: "Hybrid retinal imager using line-scanning laser ophthalmoscopy and spectral domain optical coherence tomography", OPTICS EXPRESS, vol. 14, no. 26, 22 December 2006 (2006-12-22), XP055018733, DOI: 10.1364/OE.14.012909

## Description

### TECHNICAL FIELD

The present disclosure relates generally to ophthalmic laser surgical systems, and more particularly to imaging a target within an eye and calibrating imaging devices.

### BACKGROUND

Laser vitreolysis uses laser beams to treat vitreous floaters and other retinal diseases. During a procedure, a laser beam is directed to the target, e.g., a floater, to fragment the target. The laser beam should be accurately and precisely delivered to the target to avoid damaging retinal tissues and ensure ocular safety. Known systems have attempted to improve accuracy and precision using image-guided laser beam delivery techniques. However, these techniques fail to provide satisfactory accuracy and precision in certain situations.

Reference is made to the documents US 2020/129336 A1, US 2020/038241 A1, and Milston Rebecca et al: "Vitreous floaters: Etiology, diagnostics, and management", Survey of Ophthalmology, vol. 61, no. 2, pages 211-227, ISSN: 0039-6257 which have been cited as exemplary of the background state of the art.

### BRIEF SUMMARY

The scope of the invention is in accordance with the appended claims.

The disclosed ophthalmic laser surgical system for imaging and treating a target in an eye includes an imaging system, a treatment system, a laser-OCT xy-scanner, and a computer. The eye has an eye axis that defines a z-axis, which defines xy-planes in the eye. The imaging system includes an optical coherence tomography (OCT) device and a scanning laser ophthalmoscope (SLO) device. The OCT device directs an OCT imaging beam along an imaging beam path towards the target in the eye, receives the OCT imaging beam reflected from the eye, and generates OCT images from the reflected OCT imaging beam. The SLO device directs an SLO imaging beam along an SLO imaging beam path towards the target in the eye, receives the SLO imaging beam reflected from the eye, and generates SLO images from the reflected SLO imaging beam. The SLO device includes an SLO xy-scanner that scans the SLO imaging beam in an xy-plane in the eye. The treatment system includes a laser device that directs a laser beam along a laser beam path towards the eye. The laser-OCT xy-scanner: receives the OCT imaging beam from the imaging system, directs the OCT imaging beam along the imaging beam path towards the eye, and scans the OCT imaging beam in an xy-plane in the eye; and receives the laser beam from the laser device, directs the laser beam along the laser beam path aligned with the imaging beam path towards the eye, and scans the laser beam in the xy-plane in the eye. The computer sends instructions to the OCT device and the laser device.

Embodiments may include none, one, some, or all of the following features:
* The target comprises an eye floater.
* The laser-OCT xy-scanner and the SLO xy-scanner scan concurrently.
* The OCT device determines a z-location of the target relative to the z-axis.
* The SLO device determines an xy-location of the target relative to an xy-plane.
* The SLO images comprising enface images.
* The computer tracks the target according to the plurality of SLO images.
* The computer aims the laser beam according to the OCT images and instructs the laser device to fire the laser beam.
* The SLO device generates the SLO images while the laser device fires the laser beam.
* The OCT device directs the OCT imaging beam along the OCT imaging beam path towards a calibration pattern, receives the OCT imaging beam reflected from the calibration pattern, and generates an OCT image of the calibration pattern from the reflected OCT imaging beam. The SLO device directs the SLO imaging beam along the SLO imaging beam path towards the calibration pattern, receives the SLO imaging beam reflected from the calibration pattern, and generates an SLO image of the calibration pattern from the reflected SLO imaging beam. The computer determines one or more differences between the OCT image and the SLO image of the calibration pattern, and calculates one or more correction parameters to compensate for the differences between the OCT image and the SLO image of the calibration pattern.

### BRIEF DESCRIPTION OF THE DRAWINGS

FIGURE 1 illustrates an example of an ophthalmic surgical system that can image and treat a target in an eye, according to certain embodiments;
FIGURE 2 illustrates another example of an ophthalmic surgical system that can image and treat a target in an eye, according to certain embodiments;
FIGURE 3 illustrates an example of a calibration pattern that may be used by the system(s) of FIGURES 1 and/or 2 to register OCT, laser, and/or SLO devices, according to certain embodiments;
FIGURE 4 illustrates an example of a method for imaging and treating a target in an eye that may be performed by the system(s) of FIGURES 1 and/or 2, according to certain embodiments;
FIGURE 5 illustrates an example of a method for imaging and treating a target in an eye that may be performed by the system(s) of FIGURES 1 and/or 2, according to certain embodiments; and
FIGURE 6 illustrates an example of a method for registering OCT and SLO devices that may be performed by the system(s) of FIGURES 1 and/or 2, according to certain embodiments.

### DESCRIPTION OF EXAMPLE EMBODIMENTS

Referring now to the description and drawings, example embodiments of the disclosed apparatuses, systems, and methods are shown in detail. The description and drawings are not intended to be exhaustive or otherwise limit the claims to the specific embodiments shown in the drawings and disclosed in the description. Although the drawings represent possible embodiments, the drawings are not necessarily to scale and certain features may be simplified, exaggerated, removed, or partially sectioned to better illustrate the embodiments.

Known image-guided laser beam delivery systems require registration of imaging and laser beam systems to precisely guide the beam. However, known registration techniques are complicated and often do not provide sufficient levels of accuracy. Moreover, inherent variations of the optical properties of eyes make reliable registration difficult.

Accordingly, embodiments of the systems described herein include: (1) an imaging system with an optical coherence tomography (OCT) device that directs an imaging beam towards the target to generate an image of the target; and (2) a treatment system that directs a laser beam to the target to fragment the target. The imaging and laser beams share a laser-OCT xy-scanner to automatically register the beams. The imaging system also includes a scanning laser ophthalmoscope (SLO) device with an SLO xy-scanner. The laser-OCT and SLO xy-scanners are registered using a calibration pattern.

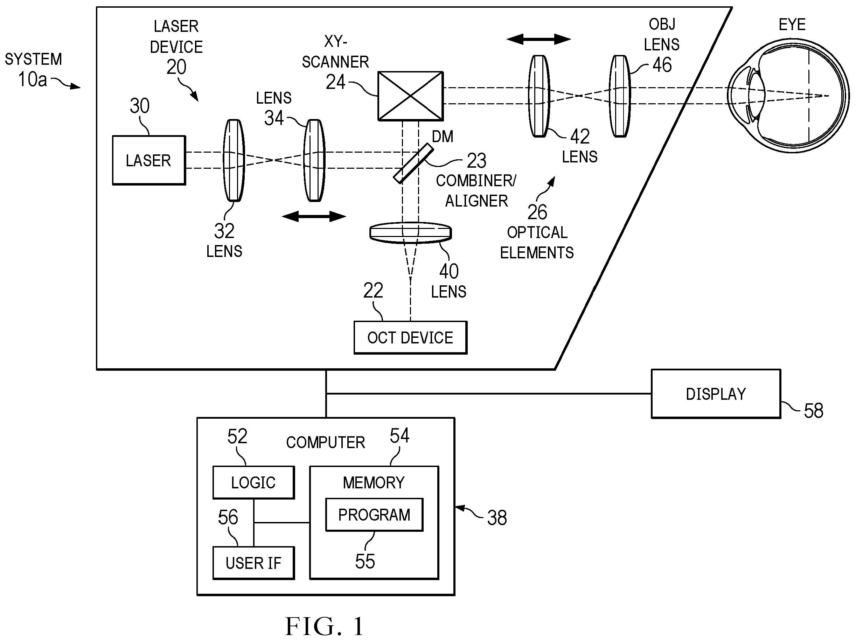

FIGURE 1 illustrates an example of an ophthalmic surgical system 10a that can image and treat a target in an eye, according to certain embodiments. In the example, the target is a vitreous eye floater or the shadow it casts on the retina. In the example, an axis of the eye (e.g., visual or optical) defines a z-axis, which in turn define x- and y-axes orthogonal to each other and to the z-axis. X- and y-axes define xy-planes within the eye. X-, y-, and z-directions and locations are relative to the x-, y-, and z-axes, respectively.

In the example, system 10a includes a treatment system (which comprises a laser device 20), an imaging system (which comprises optical coherence tomography (OCT) device 22), a beam combining and alignment device 23, a laser-OCT xy-scanner 24, optical elements 26, and a computer 38, coupled as shown. Laser device 20 includes a laser 30 and lenses 32, 34, coupled as shown. Optical components includes beam combining and alignment device 23 and lenses 32, 34, 40, 42, and 46, coupled as shown. Computer 38 includes logic 52, memory 54 (which stores a computer program 55), a user interface (IF) 56, and a display 58, coupled as shown.

As an example of operation, OCT device 22 directs an OCT imaging beam along an imaging beam path towards the eye, receives the OCT imaging beam reflected from the eye, and generates OCT images from the reflected OCT imaging beam. Laser device 20 directs a laser beam along a laser beam path towards the target. Beam combining and alignment device 23 combines and co-aligns the OCT imaging beam and the laser beam. Laser-OCT xy-scanner 24 receives the OCT imaging beam from the imaging system, directs the OCT imaging beam along the imaging beam path towards the target, and scans the OCT imaging beam in an xy-plane in the eye. Laser-OCT xy-scanner 24 also receives the laser beam from the laser device, directs the laser beam along the laser beam path co-aligned with the imaging beam path towards the target, and scans the laser beam in an xy-plane in the eye. Computer 38 sends instructions to the OCT device and the laser device.

Turning to the treatment system, laser 30 of laser device 22 generates a laser beam with any suitable wavelength, e.g., in a range from 400 nm to 2000 nm. Laser device 22 delivers laser pulses at any suitable repetition rates ranging from, but not limited to, 1 hertz (Hz) to several hundreds of kilohertz (kHz). A laser pulse may have any suitable pulse duration (e.g., ranging from, but not limited to, a nanosecond (ns) to 20 femtoseconds (fs)), any suitable pulse energy (e.g., 1 microjoule (µJ) to 10 millijoule (mJ)), and a focal point of any suitable size (e.g., ranging from 3 to 20 microns (µm), such as 7 µm). Lenses 32 and 34 are used to adjust the focus position of the laser beam within tissue, such as eye tissue.

OCT device 22 may be any suitable device that utilizes optical coherence tomography to generate images, e.g., a swept-source OCT (SS-OCT), line-field OCT, full-field OCT, or spectral-domain OCT (SD-OCT) device.

Beam combining and alignment device 23 combines and co-aligns the OCT imaging beam and the laser beam. Beam combining and alignment device 23 may be any suitable device that directs and aligns laser beams along a desired beam path, e.g., a beam combiner or a dichroic mirror (DM). In certain embodiments, beam combining and alignment device 23 may be mounted on a two-axis kinematic mount. Beam combining and alignment device 23 is positioned such that the laser and imaging beams are co-aligned, and then the mount is secured to maintain the alignment.

Laser-OCT xy-scanner 24 scans the laser and imaging beams transversely in xy-directions. Examples of scanners include a galvo scanner (e.g., a pair of galvanometrically-actuated scanner mirrors that can be tilted about mutually perpendicular axes), an electro-optical scanner (e.g., an electro-optical crystal scanner) that can electro-optically steer the beam, or an acousto-optical scanner (e.g., an acousto-optical crystal scanner) that can acousto-optically steer the beam. Laser-OCT xy-scanner 24 scans the OCT imaging beam and the laser beam in an xy-plane in the eye in any suitable manner. For example, xy-scanner 24 may scan a beam (e.g., the OCT imaging beam) in a continuous manner. As another example, xy-scanner 24 may scan a beam (e.g., the laser beam) in a stepwise manner, e.g., scanning when the laser device is not firing the laser beam and ceasing scanning when the laser device is firing the laser beam.

OCT device 22 and laser device 20 share xy-scanner 24, allowing for co-registration between the OCT imaging and treatment beams. That is, xy-scanner 24 receives the imaging beam from the imaging system and directs the imaging beam along the imaging beam path towards the target, and receives the laser beam from the laser device and directs the laser beam along the laser beam path co-aligned with the imaging beam path towards the target. The OCT imaging and treatment beams share the same path through the optics of the system and the eye, so are affected by the same optical properties and distortions along the beam path. Thus, if the imaging and treatment beams are aligned prior to xy-scanner 24, they are automatically aligned at the target location. This enables accurate and precise delivery of the laser beam to the target location identified using OCT images.

Optical elements includes beam combining and alignment device 23 and lenses 32, 34, 40, 42, and 46, coupled as shown. In general, an optical element can act on (e.g., transmit, reflect, refract, diffract, collimate, condition, shape, focus, modulate, and/or otherwise act on) a laser beam. Examples of optical elements include a lens, prism, mirror, diffractive optical element (DOE), holographic optical element (HOE), and spatial light modulator (SLM). In the example, lens 40 collimates the beam from OCT device 22 to and from beam combining and alignment device 23. Beam combining and alignment device 23 directs beams from OCT device 22 and laser device 20 to xy-scanner 24 and directs beams reflected from the eye back to OCT device 22. Lenses 32 and 34 collimate the beam from laser 30 and can also adjust the focus of a laser beam in the eye. Lens 42 and objective lens 46 collimate and focus beams toward the eye.

Computer 38 sends instructions to the OCT device and the laser device. Computer 38 may utilize computer programs 55 to perform operations. Examples of computer programs 55 include target imaging, target tracking, image processing, and target evaluation. In certain embodiments, computer 26 may use an image processing program 55 to perform image processing on an image, e.g., analyze the digital information of the image to extract information from the image. In certain embodiments, computer 26 performs image processing to analyze an image of a target or a target's shadow (i.e., "target shadow") to obtain information about the target. Localized opacities in the vitreous, such as floaters, can affect vision quality when they are in the path of light and cast a shadow onto the retina. Hence, the target shadow can provide useful information about clinical significance of the floater or other opacity, e.g., the density or contrast of the floater shadow may indicate the level of vision obstruction caused by the floater. In certain embodiments, computer 26 may use a floater shadow tracking program 55 to track floaters. Moving shadows may indicate the presence of symptomatic floaters that cause vision obstruction, rather than other vitreous structures or features that do not cause sufficient vision obstruction.

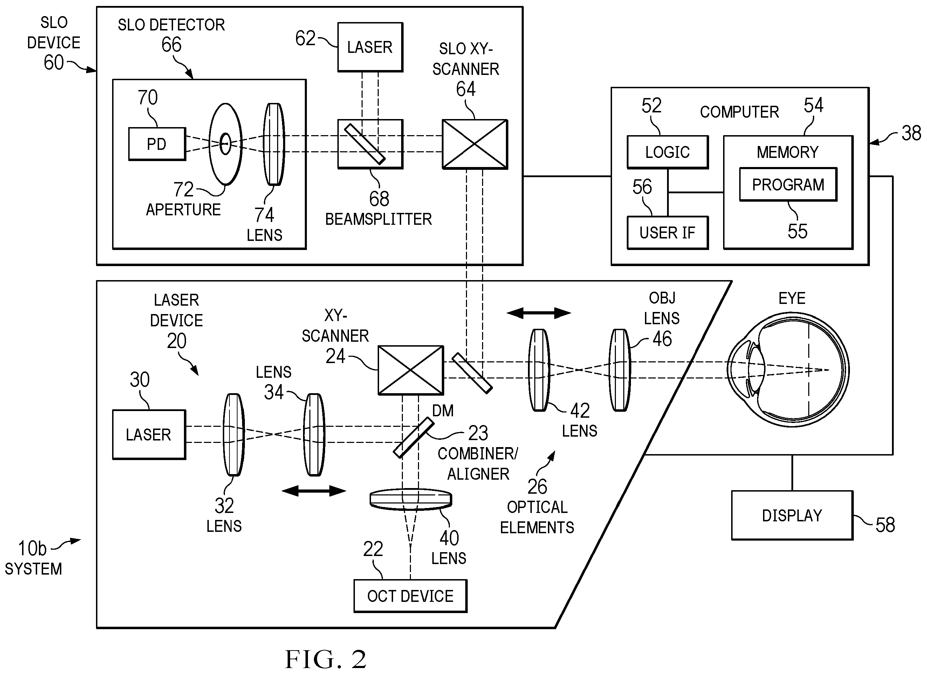

FIGURE 2 illustrates another example of an ophthalmic surgical system 10b that can image and treat a target in an eye, according to certain embodiments. In the example, system 10b is substantially similar to system 10a of FIGURE 1, except that a scanning laser ophthalmoscope (SLO) device 60 has been added to the imaging system. In the example, SLO device 60 includes a laser 62, an SLO xy-scanner 64, an SLO detector 66, and a beamsplitter 68, coupled as shown. SLO detector includes a photodetector (e.g., photodiode PD) 70, lens 72, and lens 74, coupled as shown. Analogous components of system 10b are as described with reference to FIGURE 1.

In an example of operation, OCT device 22 directs an OCT imaging beam along an OCT imaging beam path towards the eye, receives the OCT imaging beam reflected from the eye, and generates OCT images from the reflected OCT imaging beam. SLO device 60 directs an SLO imaging beam along an SLO imaging beam path towards the eye, receives the SLO imaging beam reflected from the eye, and generates SLO images from the reflected SLO imaging beam. SLO xy-scanner 64 scans the SLO imaging beam in an xy-plane in the eye. Laser device 20 directs a laser beam along a laser beam path towards the target. Laser-OCT xy-scanner 24 receives the OCT imaging beam from the imaging system, directs the OCT imaging beam along the imaging beam path towards the target, and scans the OCT imaging beam in an xy-plane in the eye. Laser-OCT xy-scanner 24 also receives the laser beam from the laser device, directs the laser beam along the laser beam path co-aligned with the imaging beam path towards the target, and scans the laser beam in an xy-plane in the eye. Computer 38 sends instructions to OCT device 22 and laser device 20.

Turning to the components, laser 62 of SLO device 60 generates any suitable SLO imaging beam, such as a laser beam with a visible or near-infrared wavelength. SLO xy-scanner 64 may be an xy-scanner as described with respect to laser-OCT xy-scanner 24. Beamsplitter 68 directs outgoing beams from laser 62 to SLO xy-scanner 64 and incoming reflected beams from the eye via SLO xy-scanner 64 to SLO detector 66. Lens 74 focuses the beam to confocal aperture 72, and the focused beam is directed to photodetector 70. Photodetector 70 detects light and generates a signal corresponding to the light. The signal may be used to generate SLO images.

OCT device 22 and SLO device 60 each provide different imaging characteristics. In certain embodiments, OCT device 22 can provide higher resolution two-dimensional (2D) and three-dimensional (3D) imaging over smaller regions. In certain embodiments, SLO device 60 can provide larger field of view (FOV) imaging and a faster 2D imaging refresh rate than that of 3D imaging by OCT device 22. For example, SLO device 60 may provide a 40 degrees or greater FOV and a 20 to 40 hertz (HZ) or greater refresh rates, while OCT device 22 may provide a 10 degrees FOV and a 10 to 20 Hz 3D refresh rate. The larger FOV of around 40 degrees allows for imaging a wider region of vitreous and retina in the eye, including the arcades to better detect floaters or other vitreous opacities that move. Moreover, target shadows may often yield higher contrast, clearer images than the targets themselves in SLO imaging. In addition, faster SLO refresh rates allow for more effective tracking of the target (e.g., by tracking the target shadow, as described above) and can yield shorter treatment times.

In certain embodiments, laser-OCT xy-scanner 24 and SLO xy-scanner 64 can scan concurrently, which allows for efficient concurrent operation of OCT device 22 and SLO device 60. For example, OCT device 22 determines the z-location of the target relative to the z-axis, while SLO device 60 determines the xy-location of the target relative to an xy-plane. As another example, OCT device 22 determines the xyz-location of the target relative to the x, y, and z-axes, while SLO device 60 provides real-time video of the eye. As another example, computer 38 can track the target according to SLO images, while aiming the laser beam according to the OCT images and instructing the laser device to fire the laser beam. As another example, SLO device 60 generates SLO images while laser device 20 fires the laser beam. As another example, SLO device 60 may detect unwanted motion of the eye during treatment and be used to shut off treatment.

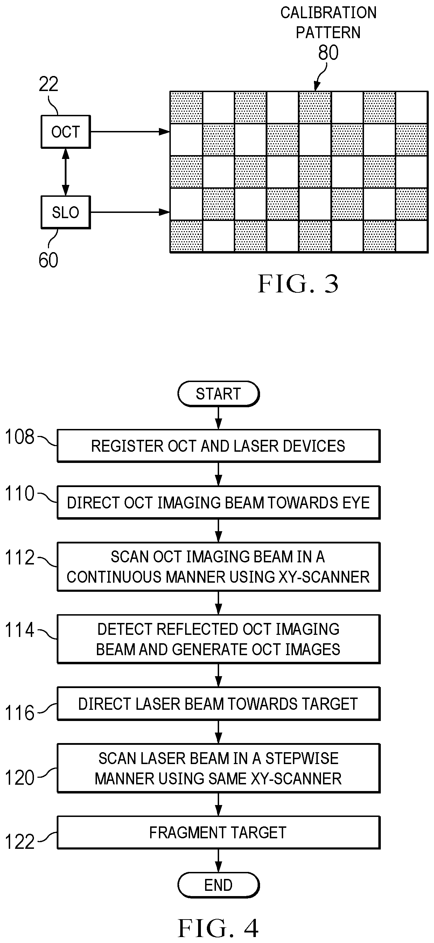

FIGURE 3 illustrates an example of a calibration pattern 80 that may be used by system(s) 10 (10a, 10b) of FIGURES 1 and/or 2 to register OCT, laser, and/or SLO devices, such as OCT device 22 and SLO device 60, according to certain embodiments. For example, xy-scanner 24 of OCT device 22 and xy-scanner 64 of SLO device 60 may be registered.

Calibration pattern 80 may be any suitable pattern that can test the xy-scanning of an imaging device. Examples of such patterns include a checkerboard, concentric circles, concentric circles with radial lines or sectors, etc. Calibration pattern 80 may be disposed upon any suitable rigid medium. In certain embodiments, the pattern may be printed on an artificial eye to provide realistic scanning of a shape that represents the eye.

In an example of operation, OCT device 22 directs the OCT imaging beam along the imaging beam path towards calibration pattern 80, receives the OCT imaging beam reflected from calibration pattern 80, and generates an OCT image of calibration pattern 80 from the reflected OCT imaging beam. SLO device 60 directs the SLO imaging beam along the imaging beam path towards calibration pattern 80, receives the SLO imaging beam reflected from calibration pattern 80, and generates an SLO images of calibration pattern 80 from the reflected SLO imaging beam.

Continuing the example of operation, computer 38 determines differences between the OCT image and the SLO image of calibration pattern 80, and calculates correction parameters that compensate for the differences to register OCT device 22 and SLO device 60. For example, computer 38 determines the position of laser-OCT xy-scanner 24 that yields the OCT beam at an xy-location of calibration pattern 80, and the position of SLO xy-scanner 64 that yields the SLO beam at the same xy-location of calibration pattern 80. Computer 38 then calculates the differences between the positions to determine correction parameters that compensate for the differences.

In the illustrated example, calibration pattern 80 is used to register OCT device 22 and SLO device 60. However, calibration pattern 80 may be used for any suitable calibration. In certain embodiments, calibration pattern 80 is used to calibrate laser device 20 and OCT device 22. In the embodiments, laser device 20 directs the laser beam towards a laser xy-location of calibration pattern 80 to yield a mark at the laser xy-location. OCT device 22 directs the OCT imaging beam towards calibration pattern 80, receives the OCT imaging beam reflected from calibration pattern 80, and generates an OCT image of the mark on calibration pattern 80 from the reflected OCT imaging beam. Computer 38 performs a registration procedure by determining differences between the OCT xy-location of the mark on the OCT image and the laser-xy-location of the mark, and calculating correction parameters to compensate for the differences.

Computer 38 may perform the registration procedure at any suitable point. For example, registration may be performed at production, after transporting the system to a new location, at the customer location, and/or periodically throughout the life of the device. Computer 38 may perform the registration procedure under any suitable conditions. For example, computer 38 performs the registration procedure while laser-OCT xy-scanner 24 is scanning in a continuous manner. As another example, computer 38 performs the registration procedure while laser-OCT xy-scanner 24 is scanning in a stepwise manner.

FIGURE 4 illustrates an example of a method for imaging and treating a target in an eye that may be performed by system(s) 10 (10a, 10b) of FIGURES 1 and/or 2, according to certain embodiments. The method starts at step 108, where OCT and laser devices are registered. In certain embodiments, to register the devices, the laser device directs a laser beam along towards an xy-location of a calibration pattern to yield a mark at the xy-location. The OCT device directs the OCT imaging beam towards the calibration pattern and generates an OCT image of the mark on the calibration pattern from the reflected OCT imaging beam. A computer may perform a registration procedure by determining differences between the OCT xy-location of the mark on the OCT image and the laser xy-location of the mark, and calculating correction parameters that compensate for the differences.

The OCT device directs an OCT imaging beam toward the eye at step 110. The xy-scanner scans the OCT imaging beam in the eye in a continuous manner at step 112. The interior of the eye reflects the OCT imaging beam. The OCT device detects the reflected OCT imaging beam and generates OCT images from the reflected beam at step 114.

The laser device directs a laser beams towards the target at step 116. The xy-scanner scans the laser beam in the eye in a stepwise manner at step 120. The xy-scanner may scan in a stepwise manner by scanning when the laser device is not firing the laser beam and ceasing scanning when the laser device is firing the laser beam. Since the OCT and laser devices share the xy-scanner, if the imaging and treatment beams are co-aligned prior to the xy-scanner, the two beams are automatically registered. (If there is a misalignment between the beams prior to the xy-scanner, a correction parameter obtained from registering the OCT and laser devices can be applied to compensate for the misalignment.) The target is fragmented at step 122.

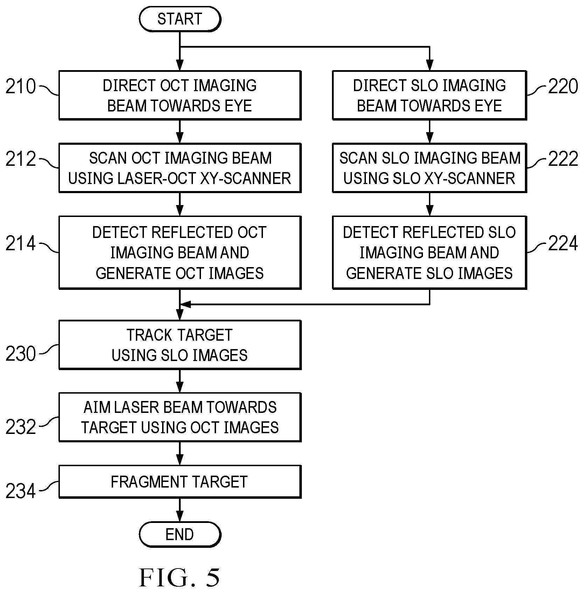

FIGURE 5 illustrates an example of a method for imaging and treating a target in an eye that may be performed by system(s) 10 (10a, 10b) of FIGURES 1 and/or 2, according to certain embodiments. The method starts at steps 210 and 220. Steps 210, 212, and 214 may be performed concurrently with steps 220, 222, and 224 to concurrently generate OCT and SLO images. The OCT and SLO devices use different scanners, which facilitates concurrent generation of the OCT and SLO images.

The OCT device directs an OCT imaging beam toward the eye at step 210. The laser-OCT xy-scanner scans the OCT imaging beam in the eye at step 212. The OCT device detects the reflected OCT imaging beam and generates OCT images from the reflected beam at step 214. The SLO device directs an SLO imaging beam toward the eye at step 220. The SLO xy-scanner scans the SLO imaging beam in the eye at step 222. The SLO device detects the reflected SLO imaging beam and generates SLO images from the reflected beam at step 224.

The computer tracks the target using the SLO images at step 230. In certain embodiments, the SLO device has a faster frame refresh rate and/or wider field of view than the OCT device, so is better able to track a moving target. The laser device aims the laser beam at the target using the OCT images at step 232. The OCT and laser devices share an xy-scanner, allowing for co-registration between the imaging and treatment beams. (If appropriate, correction parameters may be applied to compensate for a misalignment or other error between any of the components of system 10, e.g., the SLO, OCT, and/or laser device.) The target is fragmented at step 234.

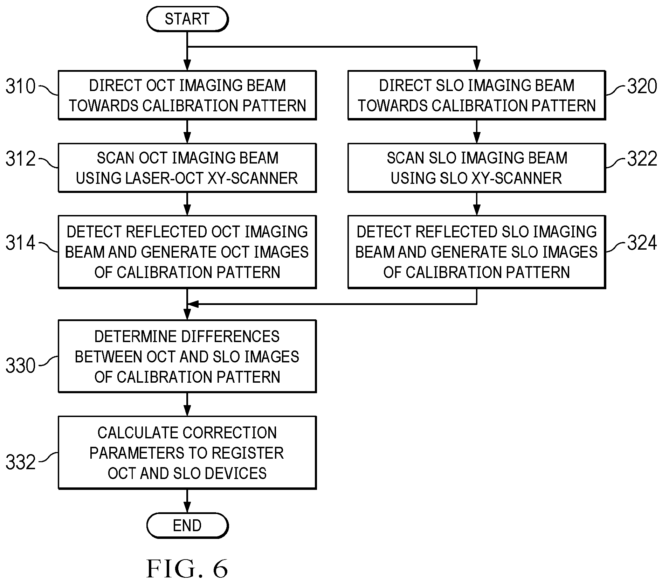

FIGURE 6 illustrates an example of a method for registering OCT and SLO devices that may be performed by system(s) 10 (10a, 10b) of FIGURES 1 and/or 2, according to certain embodiments. In certain embodiments, the OCT and SLO devices use different scanners, so the method may be performed to register the scanners of the devices. The method starts at steps 310 and 320. Steps 310, 312, and 314 may be performed concurrently with steps 320, 322, and 324 to concurrently generate OCT and SLO images of a calibration pattern.

The OCT device directs an OCT imaging beam toward the calibration pattern at step 310. The laser-OCT xy-scanner scans the OCT imaging beam across the calibration pattern at step 312. The OCT device detects the reflected OCT imaging beam and generates OCT images of the calibration pattern from the reflected beam at step 314. The SLO device directs an SLO imaging beam toward the calibration pattern at step 320. The SLO xy-scanner scans the SLO imaging beam across the calibration pattern at step 322. The SLO device detects the reflected SLO imaging beam and generates SLO images of the calibration pattern from the reflected beam at step 324.

The computer determines differences between the OCT and SLO images of the calibration pattern at step 330. The computer determines correction parameters that compensate for the differences at step 332 in order to register the OCT and SLO devices. For example, the computer determines the position of the laser-OCT xy-scanner that yields the OCT beam at an xy-location of the calibration pattern, and the position of the SLO xy-scanner that yields the SLO beam at the same xy-location of the calibration pattern. The computer calculates the differences between the positions to determine correction parameters that compensate for the differences.

A component (such as the control computer) of the systems and apparatuses disclosed herein may include an interface, logic, and/or memory, any of which may include computer hardware and/or software. An interface can receive input to the component and/or send output from the component, and is typically used to exchange information between, e.g., software, hardware, peripheral devices, users, and combinations of these. A user interface is a type of interface that a user can utilize to communicate with (e.g., send input to and/or receive output from) a computer. Examples of user interfaces include a display, Graphical User Interface (GUI), touchscreen, keyboard, mouse, gesture sensor, microphone, and speakers.

Logic can perform operations of the component. Logic may include one or more electronic devices that process data, e.g., execute instructions to generate output from input. Examples of such an electronic device include a computer, processor, microprocessor (e.g., a Central Processing Unit (CPU)), and computer chip. Logic may include computer software that encodes instructions capable of being executed by an electronic device to perform operations. Examples of computer software include a computer program, application, and operating system.

A memory can store information and may comprise tangible, computer-readable, and/or computer-executable storage medium. Examples of memory include computer memory (e.g., Random Access Memory (RAM) or Read Only Memory (ROM)), mass storage media (e.g., a hard disk), removable storage media (e.g., a Compact Disk (CD) or Digital Video or Versatile Disk (DVD)), database, network storage (e.g., a server), and/or other computer-readable media. Particular embodiments may be directed to memory encoded with computer software.

Although this disclosure has been described in terms of certain embodiments, modifications (such as changes, substitutions, additions, omissions, and/or other modifications) of the embodiments will be apparent to those skilled in the art. Accordingly, modifications may be made to the embodiments without departing from the scope of the invention. For example, modifications may be made to the systems and apparatuses disclosed herein. The components of the systems and apparatuses may be integrated or separated, or the operations of the systems and apparatuses may be performed by more, fewer, or other components, as apparent to those skilled in the art. As another example, modifications may be made to the methods disclosed herein. The methods may include more, fewer, or other steps, and the steps may be performed in any suitable order, as apparent to those skilled in the art.

The invention is defined in the following claims. Other embodiments, items, aspects, methods etc. are not a part of the invention.

## Claims

1. An ophthalmic laser surgical system for imaging and treating a target in an eye, comprising:
an imaging system (10b) comprising:
an optical coherence tomography, OCT, device (22) configured to:
direct an OCT imaging beam along an OCT imaging beam path towards the target in the eye, the eye having an eye axis, the eye axis defining a z-axis, the z-axis defining a plurality of xy-planes in the eye;
receive the OCT imaging beam reflected from the eye; and
generate a plurality of OCT images from the reflected OCT imaging beam; and
a scanning laser ophthalmoscope, SLO, device (60) configured to:
direct an SLO imaging beam along an SLO imaging beam path towards the target in the eye;
receive the SLO imaging beam reflected from the eye; and
generate a plurality of SLO images from the reflected SLO imaging beam, the SLO device comprising an SLO xy-scanner (64) configured to scan the SLO imaging beam in an xy-plane in the eye;
a treatment system comprising a laser device (20) configured to direct a laser beam along a laser beam path towards the eye; and
a laser-OCT xy-scanner (24) configured to:
receive the OCT imaging beam from the imaging system (22), direct the OCT imaging beam along the OCT imaging beam path towards the eye, and scan the OCT imaging beam in an xy-plane in the eye; and
receive the laser beam from the laser device (20), direct the laser beam along the laser beam path aligned with the OCT imaging beam path towards the eye, and scan the laser beam in the xy-plane in the eye; and
a computer (38) configured to send instructions to the OCT device (22) and the laser device (20).

2. The ophthalmic laser surgical system of claim 1, further comprising:
a beam combining and alignment device (23) configured to align the OCT imaging beam and the laser beam.

3. The ophthalmic laser surgical system of Claim 1, the target comprising an eye floater.

4. The ophthalmic laser surgical system of Claim 1, the laser-OCT xy-scanner (24) configured to scan the OCT imaging beam in the xy-plane in the eye by:
scanning the OCT imaging beam in a continuous manner.

5. The ophthalmic laser surgical system of Claim 1, the laser-OCT xy-scanner (24) configured to scan the laser beam in the xy-plane in the eye by:
scanning the laser beam in a stepwise manner by scanning when the laser device (20) is not firing the laser beam and ceasing scanning when the laser device is firing the laser beam.

6. The ophthalmic laser surgical system of Claim 1:
the laser device (20) configured to:
direct the laser beam along towards a laser xy-location of a calibration pattern to yield a mark at the laser xy-location; and
the OCT device (22) configured to:
direct the OCT imaging beam towards the calibration pattern;
receive the OCT imaging beam reflected from the calibration pattern; and
generate an OCT image of the mark on the calibration pattern from the reflected OCT imaging beam; and
the computer (38) configured to perform a registration procedure by:
determining a difference between an OCT xy-location of the mark on the OCT image and the laser-xy-location of the mark; and
calculating one or more correction parameters to compensate for the difference.

7. The ophthalmic laser surgical system of Claim 6, the computer (38) configured to perform the registration procedure while the laser-OCT xy-scanner (24) is scanning in a continuous manner.

8. The ophthalmic laser surgical system of Claim 6, the computer (38) configured to perform the registration procedure while the laser-OCT xy-scanner (24) is scanning in a stepwise manner.

9. The ophthalmic laser surgical system of Claim 2, the beam combining and alignment device (23) mounted on a two-axis kinematic mount.

10. The ophthalmic laser surgical system of Claim 2, the beam combining and alignment device (23) comprising a beam combiner or a dichroic mirror.

11. The ophthalmic laser surgical system of Claim 1, the laser-OCT xy-scanner and the SLO xy-scanner configured to scan concurrently.

12. The ophthalmic laser surgical system of Claim 1, the OCT device configured to:
determine a z-location of the target relative to the z-axis.

13. The ophthalmic laser surgical system of Claim 1, the SLO device configured to:
determine an xy-location of the target relative to an xy-plane.

## Patentansprüche

1. Ophthalmisches Laserchirurgiesystem zur Bildgebung und Behandlung eines Ziels in einem Auge, umfassend:
ein Bildgebungssystem (10b) umfassend:
eine Optische-Kohärenztomographie-, OCT-, Vorrichtung (22), ausgelegt zum:
Richten eines OCT-Bildgebungsstrahls entlang eines OCT-Bildgebungsstrahlengangs auf das Ziel in dem Auge, wobei das Auge eine Augenachse aufweist, wobei die Augenachse eine z-Achse definiert, wobei die z-Achse eine Mehrzahl von xy-Ebenen in dem Auge definiert;
Empfangen des von dem Auge reflektierten OCT-Bildgebungsstrahls; und
Erzeugen einer Mehrzahl von OCT-Bildern aus dem reflektierten OCT-Bildgebungsstrahl; und
eine Scanning-Laserophthalmoskop-, SLO-, Vorrichtung (60), ausgelegt zum:
Richten eines SLO-Bildgebungsstrahls entlang eines SLO-Bildgebungsstrahlengangs in Richtung zu dem Ziel in dem Auge;
Empfangen des von dem Auge reflektierten SLO-Bildgebungsstrahls; und
Erzeugen einer Mehrzahl von SLO-Bildern aus dem reflektierten SLO-Bildgebungsstrahl, wobei die SLO-Vorrichtung einen SLO-xy-Scanner (64) umfasst, dazu ausgelegt, den SLO-Bildgebungsstrahl in einer xy-Ebene in dem Auge zu scannen;
ein Behandlungssystem umfassend eine Laservorrichtung (20), dazu ausgelegt, einen Laserstrahl entlang eines Laserstrahlengangs in Richtung zu dem Auge zu richten; und
einen Laser-OCT-xy-Scanner (24), ausgelegt zum:
Empfangen des OCT-Bildgebungsstrahls von dem Bildgebungssystem (22), Richten des OCT-Bildgebungsstrahls entlang des OCT-Bildgebungsstrahlengangs in Richtung zu dem Auge und Scannen des OCT-Bildgebungsstrahls in einer xy-Ebene in dem Auge; und
Empfangen des Laserstrahls von der Laservorrichtung (20), Richten des Laserstrahls entlang des Laserstrahlengangs, der mit dem OCT-Bildgebungsstrahlengang ausgerichtet ist, in Richtung zu dem Auge und Scannen des Laserstrahls in der xy-Ebene in dem Auge; und
einen Computer (38), ausgelegt zum Senden von Anweisungen zu der OCT-Vorrichtung (22) und der Laservorrichtung (20).

2. Ophthalmisches Laserchirurgiesystem nach Anspruch 1, ferner umfassend:
eine Strahlkombinier- und -Ausrichtvorrichtung (23), dazu ausgelegt, den OCT-Bildgebungsstrahl und den Laserstrahl auszurichten.

3. Ophthalmisches Laserchirurgiesystem nach Anspruch 1, wobei das Ziel eine Glaskörpertrübung umfasst.

4. Ophthalmisches Laserchirurgiesystem nach Anspruch 1, wobei der Laser-OCT-xy-Scanner (24) dazu ausgelegt ist, den OCT-Bildgebungsstrahl in der xy-Ebene in dem Auge zu scannen durch:
Scannen des OCT-Bildgebungsstrahls auf kontinuierliche Weise.

5. Ophthalmisches Laserchirurgiesystem nach Anspruch 1, wobei der Laser-OCT-xy-Scanner (24) dazu ausgelegt ist, den Laserstrahl in der xy-Ebene in dem Auge zu scannen durch:
Scannen des Laserstrahls auf schrittweise Weise durch Scannen, wenn die Laservorrichtung (20) den Laserstrahl nicht abfeuert, und Absetzen von Scannen, wenn die Laservorrichtung den Laserstrahl abfeuert.

6. Ophthalmisches Laserchirurgiesystem nach Anspruch 1:
wobei die Laservorrichtung (20) ausgelegt ist zum:
Richten des Laserstrahls in Richtung zu einer Laser-xy-Position einer Kalibrierstruktur, um eine Markierung an der Laser-xy-Position zu erhalten; und
die OCT-Vorrichtung (22) ausgelegt ist zum:
Richten des OCT-Bildgebungsstrahls in Richtung zu der Kalibrierstruktur;
Empfangen des von der Kalibrierstruktur reflektierten OCT-Bildgebungsstrahls; und
Erzeugen eines OCT-Bildes der Markierung auf der Kalibrierstruktur aus dem reflektierten OCT-Bildgebungsstrahl; und
wobei der Computer (38) dazu ausgelegt ist, ein Abgleichsverfahren durchzuführen durch:
Bestimmen einer Differenz zwischen einer OCT-xy-Position der Markierung auf dem OCT-Bild und der Laser-xy-Position der Markierung; und
Berechnen eines oder mehrerer Korrekturparameter zum Kompensieren der Differenz.

7. Ophthalmisches Laserchirurgiesystem nach Anspruch 6, wobei der Computer (38) dazu ausgelegt ist, das Abgleichsverfahren durchzuführen, während der Laser-OCT-xy-Scanner (24) auf eine kontinuierliche Weise scannt.

8. Ophthalmisches Laserchirurgiesystem nach Anspruch 6, wobei der Computer (38) dazu ausgelegt ist, das Abgleichsverfahren durchzuführen, während der Laser-OCT-xy-Scanner (24) auf eine schrittweise Weise scannt.

9. Ophthalmisches Laserchirurgiesystem nach Anspruch 2, wobei die Strahlkombinier- und Ausrichtvorrichtung (23) an einer zweiachsigen kinematischen Halterung montiert ist.

10. Ophthalmisches Laserchirurgiesystem nach Anspruch 2, wobei die Strahlkombinier- und Ausrichtvorrichtung (23) einen Strahlvereiniger oder einen dichroitischen Spiegel umfasst.

11. Ophthalmisches Laserchirurgiesystem nach Anspruch 1, wobei der Laser-OCT-xy-Scanner und der SLO-xy-Scanner dazu ausgelegt sind, gleichzeitig zu scannen.

12. Ophthalmisches Laserchirurgiesystem nach Anspruch 1, wobei die OCT-Vorrichtung ausgelegt ist zum:
Bestimmen einer z-Position des Ziels relativ zu der z-Achse.

13. Ophthalmisches Laserchirurgiesystem nach Anspruch 1, wobei die SLO-Vorrichtung ausgelegt ist zum:
Bestimmen einer xy-Position des Ziels relativ zu einer xy-Ebene.

## Revendications

1. Système chirurgical ophtalmique à laser destiné à l'imagerie et au traitement d'une cible dans un œil, comprenant :
un système d'imagerie (10b) comprenant :
un dispositif de tomographie par cohérence optique, TCO, (22) configuré pour :
diriger un faisceau d'imagerie TCO le long d'un chemin du faisceau d'imagerie TCO dans l'œil, l'œil ayant un axe d'œil, l'axe d'œil définissant un axe z, l'axe z définissant une pluralité de plans xy dans l'œil ;
recevoir le faisceau d'imagerie TCO réfléchi par l'œil ;
générer une pluralité d'images TCO à partir du faisceau d'imagerie TCO réfléchi ; et
un dispositif d'ophtalmoscope à laser de balayage, SLO (60) configuré pour :
diriger un faisceau d'imagerie SLO le long d'un chemin du faisceau d'imagerie SLO vers la cible dans l'œil ;
recevoir le faisceau d'imagerie SLO réfléchi par l'œil ; et
générer une pluralité d'images SLO à partir du faisceau d'imagerie SLO réfléchi, le dispositif SLO comprenant un scanner xy SLO (64) configuré pour balayer le faisceau d'imagerie SLO dans un plan xy dans l'œil ;
un système de traitement comprenant un dispositif laser (20) configuré pour diriger un faisceau laser le long d'un chemin de faisceau laser vers l'œil ; et
un scanner xy laser-TCO (24) configuré pour :
recevoir le faisceau d'imagerie TCO du système d'imagerie (22), diriger le faisceau d'imagerie TCO le long du chemin du faisceau d'imagerie TCO vers l'œil, et balayer le faisceau d'imagerie TCO dans un plan xy dans l'œil ; et
recevoir le faisceau laser du dispositif laser (20), diriger le faisceau laser le long du chemin du faisceau laser aligné sur le chemin du faisceau d'imagerie TCO vers l'œil, et balayer le faisceau laser dans le plan xy dans l'œil ; et
un ordinateur (38) configuré pour envoyer des instructions au dispositif TCO (22) et au dispositif laser (20).

2. Système chirurgical ophtalmique à laser selon la revendication 1, comprenant en outre :
un dispositif de combinaison et d'alignement de faisceaux (23) configuré pour aligner le faisceau d'imagerie TCO et le faisceau laser.

3. Système chirurgical ophtalmique à laser selon la revendication 1, la cible comprenant un corps flottant d'un œil.

4. Système chirurgical ophtalmique à laser selon la revendication 1, le scanner xy laser-TCO (24) étant configuré pour balayer le faisceau d'imagerie TCO dans le plan xy dans l'œil par :
balayage du faisceau d'imagerie TCO de manière continue.

5. Système chirurgical ophtalmique à laser selon la revendication 1, le scanner xy laser-TCO (24) étant configuré pour balayer le faisceau laser dans le plan xy dans l'œil par :
balayage progressif du faisceau laser par balayage lorsque le dispositif laser (20) ne déclenche pas le faisceau laser et arrêt du balayage lorsque le dispositif laser déclenche le faisceau laser.

6. Système chirurgical ophtalmique à laser selon la revendication 1 :
le dispositif laser (20) étant configuré pour :
diriger le faisceau laser le long d'un emplacement xy de laser d'un motif d'étalonnage pour obtenir un repère à l'emplacement xy de laser ; et
le dispositif TCO (22) étant configuré pour :
diriger le faisceau d'imagerie TCO vers le motif d'étalonnage ;
recevoir le faisceau d'imagerie TCO réfléchi par le motif d'étalonnage ; et
générer une image TCO du repère sur le motif d'étalonnage à partir du faisceau d'imagerie TCO réfléchi ; et
l'ordinateur (38) étant configuré pour réaliser une procédure de recalage par :
détermination d'une différence entre un emplacement xy TCO du repère sur l'image TCO et l'emplacement xy laser du repère ; et
calcul d'un ou plusieurs paramètres de correction pour compenser la différence.

7. Système chirurgical ophtalmique à laser selon la revendication 6, l'ordinateur (38) étant configuré pour exécuter la procédure de recalage pendant que le scanner xy laser-TCO (24) balaye de manière continue.

8. Système chirurgical ophtalmique à laser selon la revendication 6, l'ordinateur (38) étant configuré pour exécuter la procédure de recalage pendant que le scanner xy laser-TCO (24) balaye de manière progressive.

9. Système chirurgical ophtalmique à laser selon la revendication 2, le dispositif de combinaison et d'alignement de faisceaux (23) étant monté sur un support cinématique à deux axes.

10. Système chirurgical ophtalmique à laser selon la revendication 2, le dispositif de combinaison et d'alignement de faisceaux (23) comprenant un combineur de faisceaux ou un miroir dichroïque.

11. Système chirurgical ophtalmique à laser selon la revendication 1, le scanner xy laser-TCO et le scanner xy SLO étant configurés pour balayer simultanément.

12. Système chirurgical ophtalmique à laser selon la revendication 1, le dispositif TCO étant configuré pour : déterminer un emplacement z de la cible par rapport à l'axe z.

13. Système chirurgical ophtalmique à laser selon la revendication 1, le dispositif SLO étant configuré pour : déterminer un emplacement xy de la cible par rapport à un plan xy.
